# EUROPEAN PATENT APPLICATION

(11) **EP 4 450 109 A1**
(43) Date of publication of application: **23.10.2024**
(21) Application number: 24171045.8
(22) Date of filing: 18.04.2024
(51) Int. Cl.: A61M 25/00, A61M 25/10, A61M 25/06

(54) **EXPANDABLE, INFLATABLE INTRODUCER SHEATH**

(30) Priority: 18.04.2023 US 202363496902 P; 11.04.2024 US 202418632462
(71) Applicant: Medtronic, Inc., Minneapolis, Minnesota 55432 (US)
(72) Inventor: Farrell, Timothy Desmond, Galway (IE)
(74) Representative: Zimmermann & Partner Patentanwälte mbB

(57) **Abstract**

An expandable introducer (100) includes a hub (106) and an introducer sheath (110) extending distally from the hub. The introducer sheath includes a balloon (111) having an interior surface (116) defining a central lumen (122) of the introducer sheath, an exterior surface (118), and an inner area (120) between the interior surface and the exterior surface. The introducer sheath includes a folded configuration and an unfolded configuration. The introducer sheath is configured to be advanced into a vessel of a patient in the folded configuration. The balloon may be partially inflated prior to advancement of a delivery device (460) through the introducer sheath such that the delivery device displaces inflation fluid in the partially inflated balloon. The introducer sheath may be unfolded by inflation of the balloon or by advancement of the delivery device through the introducer sheath. The balloon is configured to be fully inflated prior to retraction of the delivery device through the introducer sheath.

## Description

### CROSS-REFERENCE TO RELATED APPLICATIONS

This application claims the benefit of U.S. Provisional Application No. 63/496,902, filed April 18, 2023, and U.S. Patent Application No. 18/632,462, filed April 11, 2024 which are incorporated by reference herein in their entirety.

### FIELD OF THE INVENTION

The present invention relates to an introducer device for providing percutaneous access to a patient's vasculature for a transcatheter device delivery system, and, more particularly to an expandable introducer device having an expandable, inflatable introducer sheath for accommodating passage of the transcatheter device delivery system therethrough.

### BACKGROUND

An introducer device is used to provide percutaneous access to the vascular system of a patient and functions to permit the introduction and positioning of various minimally invasive delivery devices within the patient's vasculature. In general, having the smallest insertion profile is the most desirable and leads to less complications, less trauma, and improved patient outcomes. Some delivery devices, however, are large and require large sheaths to accommodate and help deliver them to the desired site within the body.

### BRIEF SUMMARY OF THE INVENTION

In accordance with a first example hereof, a method of delivering a prosthesis within a vasculature of a patient includes advancing an introducer sheath of an expandable introducer into a vessel of the patient, the introducer sheath being in a folded configuration, the introducer sheath including a balloon having an interior surface defining a central lumen of the introducer sheath, an exterior surface, and an inner area between the interior surface and the exterior surface; partially inflating the balloon of the balloon introducer sheath by introducing inflation fluid into the inner area of the balloon; and advancing a delivery device through the central lumen of the introducer sheath, wherein advancing the delivery device cause the inflation fluid within the balloon to displace.

In a second example, the method according to any of the previous or subsequent examples herein further comprises, after the delivery device has been advanced through the central lumen of the introducer sheath, fully inflating the balloon of the introducer sheath; and with the balloon fully inflated, retracting the delivery device proximally through the central lumen of the introducer sheath.

In a third example, the method according to any of the previous or subsequent examples herein further comprises, inserting a dilator into the expandable introducer prior to advancing the introducer sheath into the patient's vessel, wherein advancing the introducer sheath into the patient's vessel comprises advancing the introducer sheath and a dilator shaft of the dilator into the patient's vessel.

In a fourth example, the method according to any of the previous or subsequent examples herein further comprises removing the dilator from the expandable introducer prior to advancing the delivery device through the central lumen of the introducer sheath.

In a fifth example, in the method according to any of the previous or subsequent examples herein, inflating the balloon causes the introducer sheath to unfold to an unfolded configuration.

In a sixth example, in the method according to any of the previous or subsequent examples herein, advancing the delivery device through the central lumen of the introducer sheath causes the introducer sheath to unfold to an unfolded configuration.

In a seventh example, a method of delivering a prosthesis within a vasculature of a patient comprises: advancing an introducer sheath of an expandable introducer into a vessel of the patient, the introducer sheath being in a folded configuration, the introducer sheath including a balloon having an interior surface defining a central lumen of the introducer sheath, an exterior surface, and an inner area between the interior surface and the exterior surface; and advancing a delivery device through the central lumen of the introducer sheath, wherein advancing the delivery device causes introducer sheath to unfold to an unfolded configuration.

In an eighth example, the method according to any of the previous or subsequent examples herein further comprises, after the delivery device has been advanced through the central lumen of the introducer sheath, fully inflating the balloon of the introducer sheath; and with the balloon fully inflated, retracting the delivery device proximally through the central lumen of the introducer sheath.

In a ninth example, the method according to any of the previous or subsequent examples herein further comprises inserting a dilator into the expandable introducer prior to advancing the introducer sheath into the patient's vessel, wherein advancing the introducer sheath into the patient's vessel comprises advancing the introducer sheath and a dilator shaft of the dilator into the patient's vessel.

In a tenth example, the method according to any of the previous or subsequent examples herein further comprises removing the dilator from the expandable introducer prior to advancing the delivery device through the central lumen of the introducer sheath.

In an eleventh example, an expandable introducer for delivering a medical device includes an introducer hub, and an introducer sheath coupled to the introducer hub and extending distally therefrom, wherein the introducer sheath includes a balloon having an interior surface defining a central lumen of the introducer sheath, an exterior surface, and an inner area between the interior surface and the exterior surface, the inner area configured to be inflated, wherein at least a portion of the introducer sheath includes a fold such that the introducer sheath includes a folded configuration and an unfolded configuration, wherein the balloon introducer sheath transitions from the folded configuration to the unfolded configuration in response to a delivery device being advanced therethrough of to the balloon introducer sheath being inflated.

In a twelfth example, in the expandable introducer according to any of the previous or subsequent examples herein, the balloon includes one or more ridges on the exterior surface thereof.

In a thirteenth example, in the expandable introducer according to any of the previous or subsequent examples herein, the introducer sheath is configured to be loaded onto a dilator in the folded configuration prior to insertion within a vessel of a patient.

In a fourteenth example, in the expandable introducer according to any of the previous or subsequent examples herein, the balloon of the introducer sheath is configured to be partially inflated prior to advancing a delivery device therethrough.

In a fifteenth example, in the expandable introducer according to any of the previous or subsequent examples herein, with the balloon partially inflated, wherein advancement of the delivery device through the balloon introducer sheath causes inflation fluid within the balloon introducer sheath to displace.

In a sixteenth example, in the expandable introducer according to any of the previous or subsequent examples herein, the balloon is configured to be fully inflated prior to retraction of the delivery device from the introducer sheath.

In a seventeenth example, in the expandable introducer according to any of the previous or subsequent examples herein, an outer diameter of the introducer sheath in the unfolded configuration is greater than an outer diameter of the balloon introducer sheath in the folded configuration.

In an eighteenth example, in the expandable introducer according to any of the previous or subsequent examples herein, the expandable introducer further includes a spine coupled to the balloon introducer sheath.

In a nineteenth example, in the expandable introducer according to any of the previous or subsequent examples herein, the spine is a rigid polymer.

In a twentieth example, in the expandable introducer according to any of the previous or subsequent examples herein, the spine runs longitudinally along the balloon.

In a twenty-first example, in the expandable introducer according to any of the previous or subsequent examples herein, wherein the spine is disposed opposite the fold.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

The foregoing and other features and advantages of the present disclosure will be apparent from the following description of embodiments hereof as illustrated in the accompanying drawings. The accompanying drawings, which are incorporated herein and form a part of the specification, further serve to explain the principles of the present disclosure and to enable a person skilled in the pertinent art to make and use the embodiments of the present disclosure. The drawings may not be to scale.
FIG. 1 illustrates a side-view of an exemplary expandable introducer according to embodiments hereof.
FIG. 2A illustrates a schematic side-view cross-section of an introducer sheath disposed on a mandrel according to embodiments hereof.
FIG. 2B illustrates a schematic side-view cross-section of an introducer sheath disposed on a tapered mandrel according to embodiments hereof.
FIG. 2C illustrates a cross-section taken at line C-C of FIGS. 2A and 2B.
FIG. 2D illustrates a cross-section taken at line D-D of FIG. 2B.
FIG. 3 illustrates a side-view of an exemplary dilator according to embodiments hereof.
FIG. 4 is a block diagram illustrating a method of delivering a device within a vessel of a patient using the expandable introducer according to embodiments hereof.
FIG. 4A illustrates a step in the method of FIG. 3, illustrating the balloon introducer sheath in a folded, uninflated configuration within the vessel of a patient according to embodiments hereof.
FIG. 4B illustrates a step in the method of FIG. 3, illustrating the balloon introducer sheath in an unfolded, partially inflated configuration within the vessel of the patient according to embodiments hereof.
FIG. 4C illustrates a step in the method of FIG. 3, illustrating a delivery device being advanced through the unfolded, partially inflated balloon introducer sheath in the vessel of the patient according to embodiments hereof.
FIG. 4D illustrates a close-up view of the delivery device being advanced through the unfolded, partially inflated balloon introducer sheath of FIG. 4C.
FIG. 4E illustrates a step in the method of FIG. 3, illustrating a delivery device being advanced through the folded, uninflated balloon introducer sheath in the vessel of the patient according to embodiments hereof.
FIG. 4F illustrates a step in the method of FIG. 3, illustrating a delivery device being proximally retracted through the fully inflated balloon introducer sheath in the vessel of the patient according to embodiments hereof.
FIG. 5 illustrates a side-view of an exemplary expandable introducer according to embodiments hereof.
FIG. 6 illustrates a schematic longitudinal cross-sectional view of the expandable introducer of FIG. 5 with the introducer sheath thereof in an uninflated configuration.
FIG. 7 illustrates a schematic longitudinal cross-sectional view of the expandable introducer of FIG. 5 with the introducer sheath thereof in an inflated configuration.
FIG. 8 illustrates a schematic longitudinal cross-sectional view of the expandable introducer sheath of FIG, 5 with a dilator extending therethrough an inserted into a vessel of a patient according to embodiments hereof.
FIG. 9A illustrates a schematic longitudinal cross-sectional view of the expandable introducer of FIG. 5 with the dilator removed and the introducer sheath thereof in a partially inflated configuration.
FIG. 9B illustrates a schematic cross-sectional view taken along line 9-9 of FIG. 9A.
FIG. 10A illustrates a schematic cross-sectional view of the expandable introducer of FIG. 5 with the introducer sheath in a partially inflated configuration and a delivery device extending therethrough.
FIG. 10B illustrated a schematic cross-sectional view taken along line 10-10 of FIG. 10A.

### DETAILED DESCRIPTION

It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single device or component for purposes of clarity, it should be understood that the techniques of this disclosure may be performed by a combination of devices or components associated with, for example, a delivery system. The following detailed description is merely exemplary in nature and is not intended to limit the invention of the application and uses of the invention. Furthermore, there is no intention to be bound by any expressed or implied theory presented in the preceding field of the invention, background, summary or the following detailed description.

As used in this specification, the singular forms "a", "an" and "the" specifically also encompass the plural forms of the terms to which they refer, unless the content clearly dictates otherwise. The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 5%. It should be understood that use of the term "about" also includes the specifically recited number of value.

The terms "proximal" and "distal" herein when used with respect to a delivery system are used with reference to the clinician using the devices. Therefore, "proximal" and "proximally" mean in the direction toward the clinician, and "distal" and "distally" mean in the direction away from the clinician.

As used herein, the term "generally" and "substantially" mean approximately. When used to describe angles such as "substantially parallel" or "substantially perpendicular" the term "substantially" means within 10 degrees of the angle. When used to describe shapes such as "substantially" or "generally" cylindrical or "substantially" or "generally" tube-shaped or "generally" or "substantially" conical, the terms mean that the shape would appear cylindrical or tube-shaped or conical to a person of ordinary skill in the art viewing the shape with a naked eye.

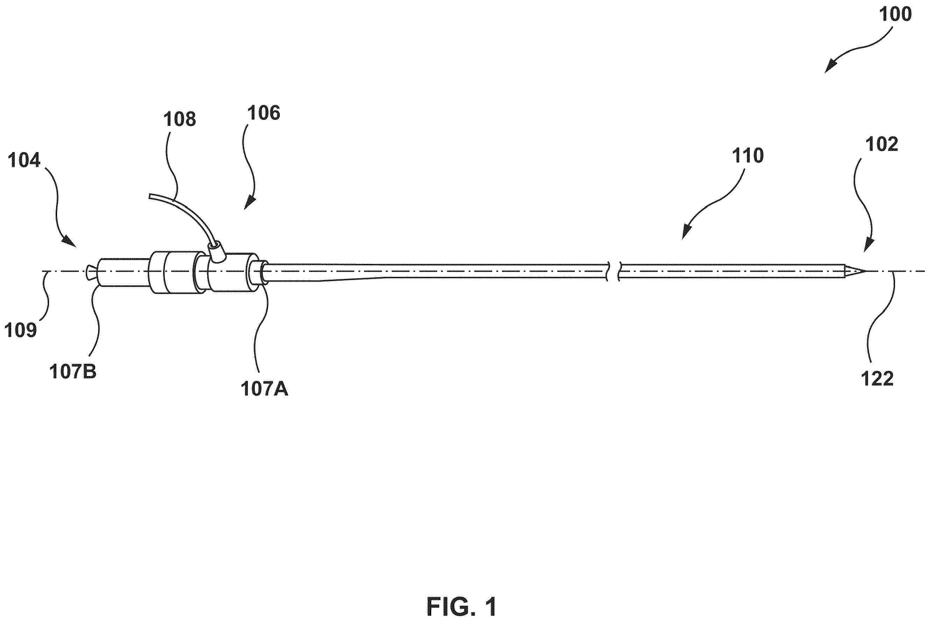

FIG. 1 shows an expandable introducer 100 according to embodiments hereof. The expandable introducer 100 includes an introducer hub 106 and an introducer sheath 110. The expandable introducer 100 includes a distal end 102 and a proximal end 104. The introducer sheath 110 is coupled to a distal end 107A of the introducer hub 106 and extends distally therefrom.

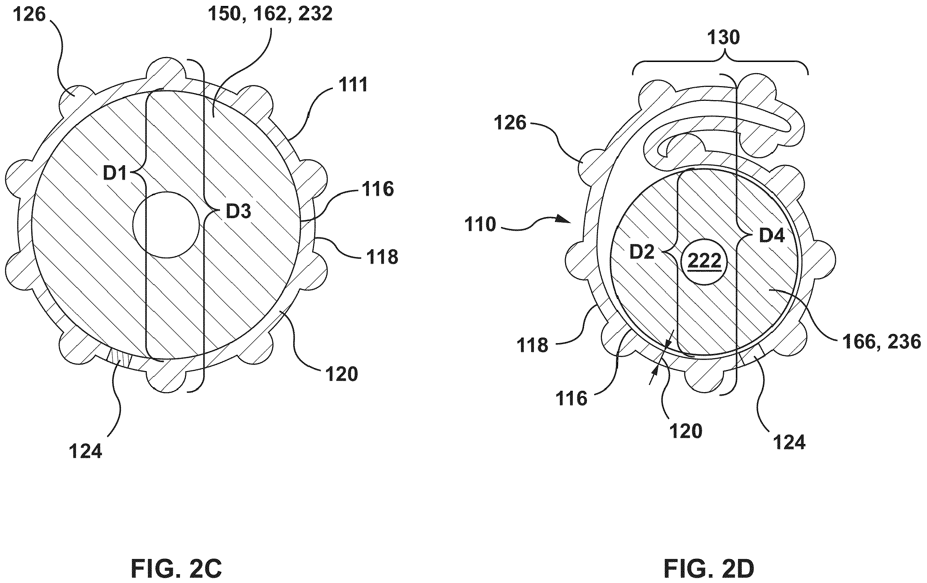

The introducer hub 106 includes the distal end 107A, a proximal end 107B, and a passageway 109 extending from the proximal end 107B to the distal end 107A of the introducer hub 106. The passageway 109 is sized and shaped to accommodate a delivery system therethrough, as described in more detail below. The introducer hub 106 may further include a side port or luer 108 disposed on an outer surface of the introducer hub 106 that is configured to inflate the introducer sheath 110 when in use, which is described in further detail below. In other words, the luer 108 may be utilized for infusing saline or other inflation fluids into the balloon introducer sheath 110, as shown and described with respect to FIG. 2C. The introducer hub 106 may further include a seal (not shown) disposed within the passageway 109 to seal around a dilator shaft of a dilator (not shown) or the delivery system, and prevent blood flow out of a patient's vessel when the introducer 100 is in use.

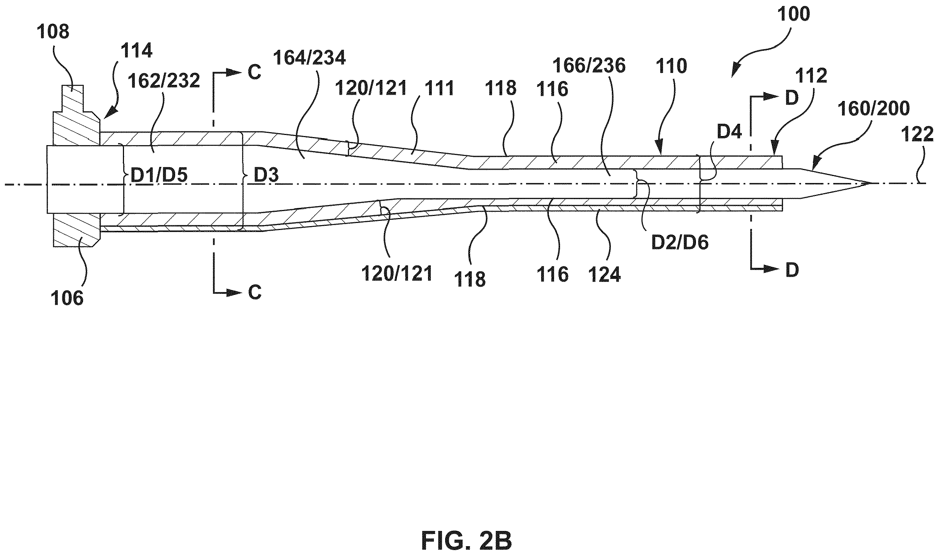

The introducer sheath 110 includes a substantially tubular-shaped balloon 111 defining a lumen 122 of the introducer sheath 110. The introducer sheath 110 may also be referred to as a balloon introducer sheath. The balloon 111 includes a distal end 112, a proximal end 114, an interior surface 116 and an exterior surface 118, as best shown in FIG. 2B, which also define the distal end, proximal end, interior surface, and exterior surface of the introducer sheath 110. A space between the interior surface 116 and the exterior surface 118 of the balloon 111 defines an inner area 120 of the balloon 111, which defines a wall 121 of the introducer sheath 110. The inner area 120 of the balloon 111 may be inflated to expand the wall 121 of the introducer sheath 110, *i.e.* increase the thickness of the wall 121 of the introducer sheath 110, as described in further detail below. The interior surface 116 of the balloon 111 defines the lumen 122 of the introducer sheath 110 that extends from the distal end 112 to the proximal end 114 of the introducer sheath 110. The lumen 122 is sized and shaped to accommodate a dilator 200 or delivery system therethrough, as explained in further detail below.

At least a portion of the balloon 111, and hence the introducer sheath 110, is configured to be folded into a folded configuration during insertion within a vessel of a patient and can be unfolded to an unfolded configuration via inflation of the balloon 111, as explained in further detail below. The introducer sheath 110 may have a longitudinal length of about 370 mm and may comprise low friction flexible materials such as, but not limited to, polyvinyl chloride (PVC), polyethylene terephthalate (PET), and/or any other materials known to those skilled in the art.

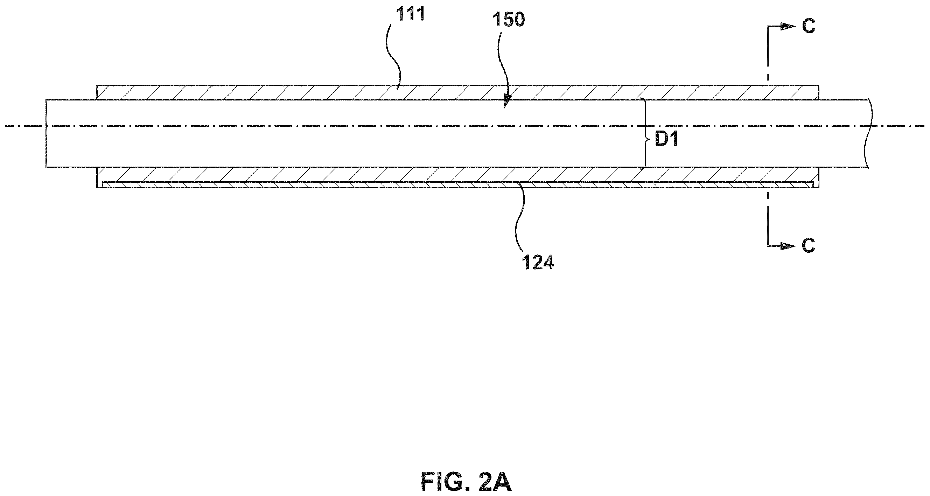

FIG. 2A shows a schematic cross-section of the introducer sheath 110 of the expandable introducer 100 disposed on a mandrel 150. The mandrel 150 may be used during processing of the introducer sheath 110. Thus, for example, the balloon 111 may be placed formed around the mandrel 150 such as to create the lumen 122 of the introducer sheath 110. In an embodiment, the mandrel 150 may be substantially cylindrical and may have a first diameter D1. In an embodiment, a spine 124 may be fused to the exterior surface 118 of the balloon 111. The spine 124 may be a rigid (*i.e*., high durometer) polymer material, such as, but not limited to, 63-72D polyether block amide (PEBAX), or other similar materials. The spine 124 may provide column strength for removal of a delivery system from the introducer 100, as described in more detail below. The spine 124 may extend in a substantially straight line longitudinally from the proximal end 114 to the distal end 112 of the introducer sheath 110, as shown in FIG. 2A. However, this is not meant to be limiting, as the spine 124 may be any shape or configuration known to those skilled in the art.

FIG. 2B shows the balloon 111 disposed on a tapered mandrel 160. The tapered mandrel 160 includes a first portion 162, a tapered second portion 164, and a third portion 166. The tapered second portion 164 is disposed between the first portion 162 and the third portion 166. The first portion 162 of the mandrel 160 may be substantially cylindrical and have a first diameter D1 substantially equal to the first diameter D1 of the mandrel 150. The third portion 166 may be substantially cylindrical and have a second diameter D2 that is smaller than the first diameter D1. The tapered second portion 164 has is tapered from the first diameter D1 where the tapered second portion 164 meets the first portion 162 to the second diameter D2 where the tapered second portion 164 meets the third portion 166. Although FIG. 2B is describes the tapered mandrel 160, a dilator 200 may have the same shape, as described in further detail below. Described in more detail below, the tapered dilator 200 may include a first portion 232 similar to the first portion 162 of the mandrel 160, a second tapered portion 234 similar to the second tapered portion 164 of the mandrel 160, and a third portion 236 similar to the third portion 166 of the mandrel 160.

FIG. 2C shows a schematic cross section taken along line C-C of FIGS. 2A and 2B. As can be seen, the mandrel shown in FIG. 2C is the mandrel 150 of FIG. 2A or the first portion 162 of the mandrel 160 of FIG. 2B. Thus, the mandrel 150/first portion 162 has the first diameter D1. The balloon 111 disposed over the mandrel 150/first portion 162 has a third outer diameter D3. The third outer diameter D3 is with the balloon 111 uninflated. As can be seen in FIG. 2C, in some embodiments, the exterior surface 118 of the balloon 111 of the introducer sheath 110 may include a plurality of ridges or protrusions 126 extending outwardly from the exterior surface 118. The ridges 126 are configured to prevent sealing the balloon 111 to the vessel of the patient. The ridges 126 are also configured to reduce the area of contact between the balloon 111 and the vessel of the patient. This configuration reduces friction between the balloon 111 and the vessel of the patient in order to reduce the necessary insertion force when advancing the introducer sheath 110 within the vessel of the patient. In the embodiment shown, the ridges 126 are spaced equidistantly apart around the exterior surface 118 of the balloon 111. The ridges 126 can be longitudinal protrusions that extend from the distal end 112 to the proximal end 114 of the balloon introducer sheath 110 and have substantially semi-circle shaped cross-sections, as shown in FIG. 2C. However, this is not meant to be limiting, as the ridges 126 may be any shape or configuration known to those skilled in the art.

FIG. 2D shows a cross-section of the balloon 111 and the third portion 166 of the mandrel 160 or the third portion 236 of the dilator 200 taken at line D-D of FIG. 2B. Thus, the third portion 166/236 of the mandrel 160/dilator 200 has the second diameter D2 described above, which is smaller than the first diameter D1 described above. The balloon 111 at the third portion 166/236 is folded into a folded configuration. In the embodiment shown, the third portion 166/236 includes a single fold 130, but this is not meant to be limiting. In the folded configuration, the balloon 111 at the third portion 166/236 of the mandrel 160/dilator 200 has an outer diameter D4 which is smaller than the outer diameter D3 described above. The outer diameter D2 in the folded configuration may be between about 5.25 mm and about 6.3 mm.

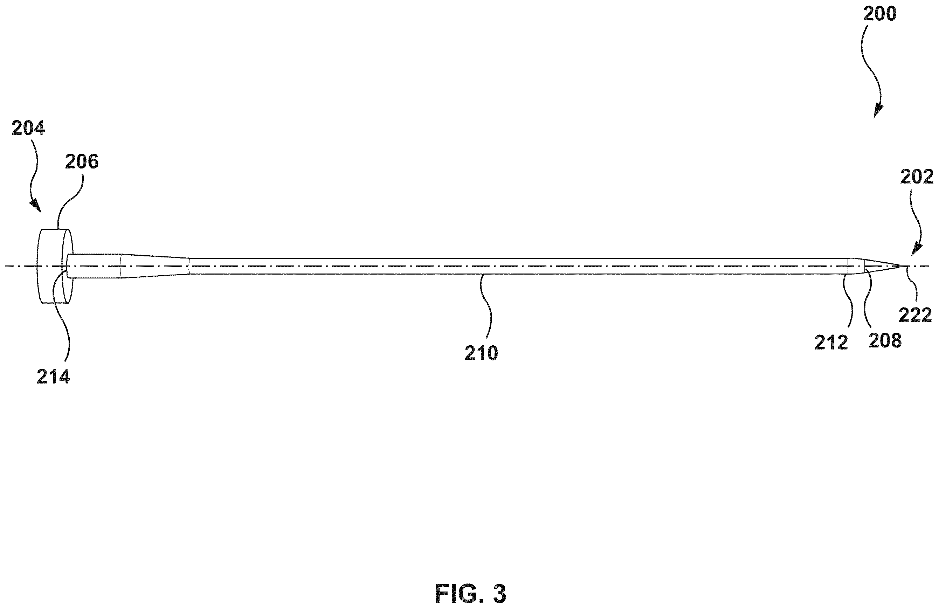

FIG. 3 shows an exemplary dilator 200 according to embodiments hereof. The dilator 200 includes a distal end 202, a proximal end 204, a dilator hub 206, a dilator tip 208 and a dilator shaft 210. A proximal end 214 of the dilator shaft 210 is coupled to the dilator hub 206 and extends distally therefrom. The dilator tip 208 is coupled to a distal end 212 of the dilator shaft 210 and extends distally therefrom. A guidewire lumen 222 extends an entire longitudinal length of the dilator 200, extending through the dilator hub 206, the dilator shaft 210 and the dilator tip 208 from the distal end 202 to the proximal end 204 of the dilator 200. The dilator tip 208 is substantially conical-shaped such that it tapers in a distal direction. Accordingly, an outer diameter of a proximal end of the dilator tip 208 is greater than an outer diameter of a distal end of the dilator tip 208. The dilator 200 may have a longitudinal length, from the distal end 202 to the proximal end 204, of about 400 mm to about 450 mm and may comprise high density polyethylene (HDPE), low density polyethylene (LDPE) and/or any other materials known to those skilled in the art.

As noted briefly above, the dilator shaft 210 may be tapered in the distal direction, as shown in FIG. 2B. This the dilator shaft 210 may include a first portion 232, a tapered second portion 234, and a third portion 236. The tapered second portion 234 is disposed between the first portion 232 and the third portion 236. The first portion 232 of the dilator shaft 210 may be substantially cylindrical and have a fifth diameter D5. The third portion 236 may be substantially cylindrical and have a sixth diameter D6 that is smaller than the fifth diameter D5. The tapered second portion 234 is tapered from the fifth diameter D5 where the tapered second portion 234 meets the first portion 232 to the sixth diameter D6 where the tapered second portion 234 meets the third portion 236. In an embodiment, the fifth diameter D5 may be substantially equal to the first diameter D1 of the mandrel 160, and the sixth diameter D6 may be substantially equal to the second diameter D2 of the mandrel 160.

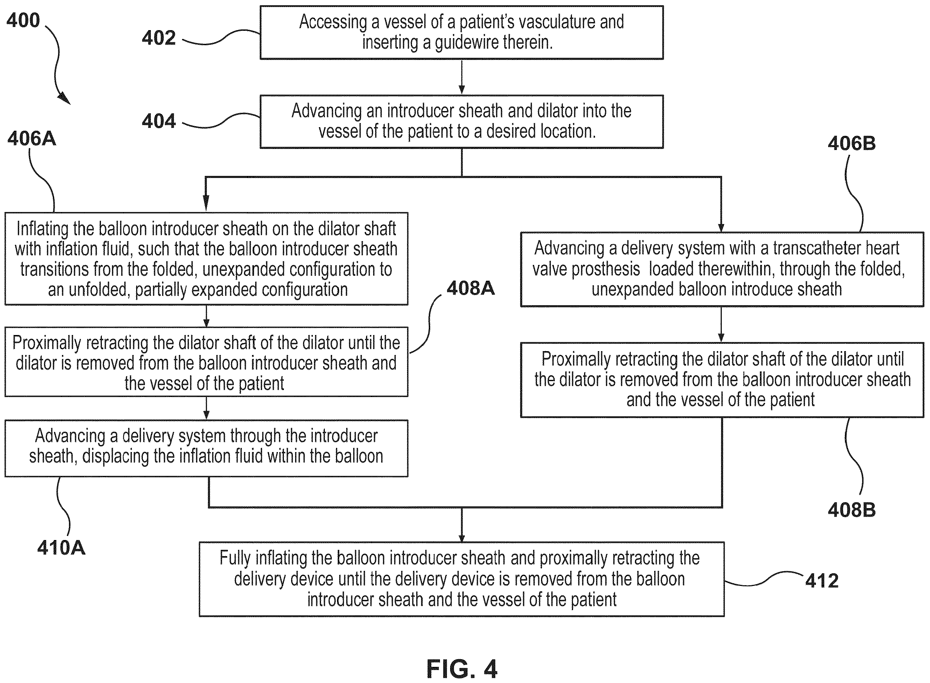

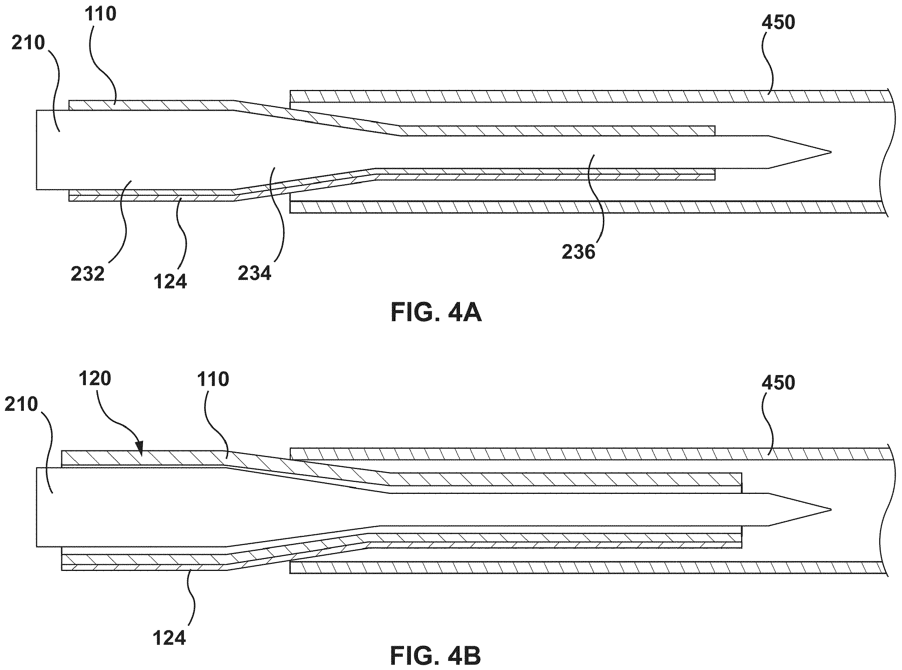

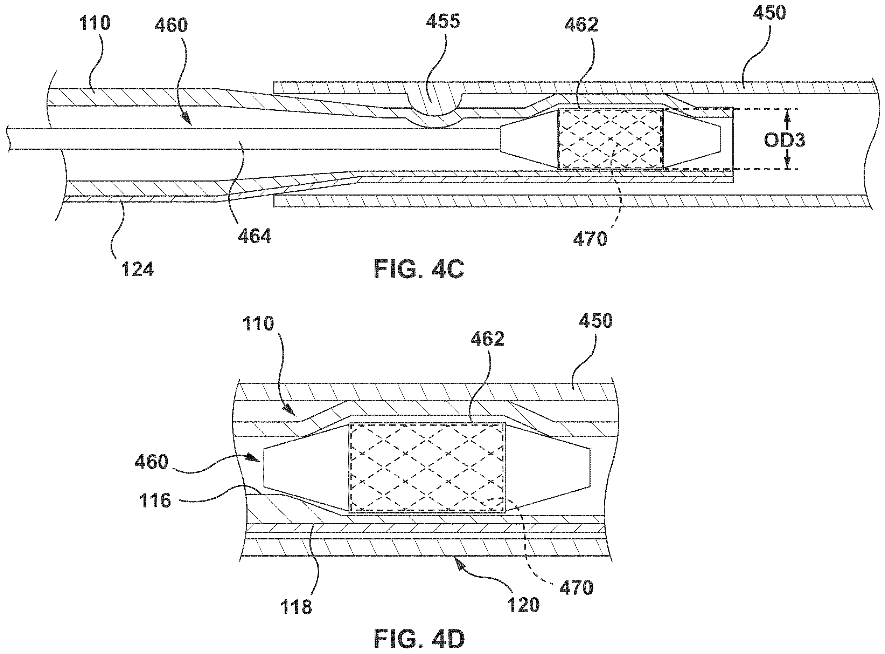

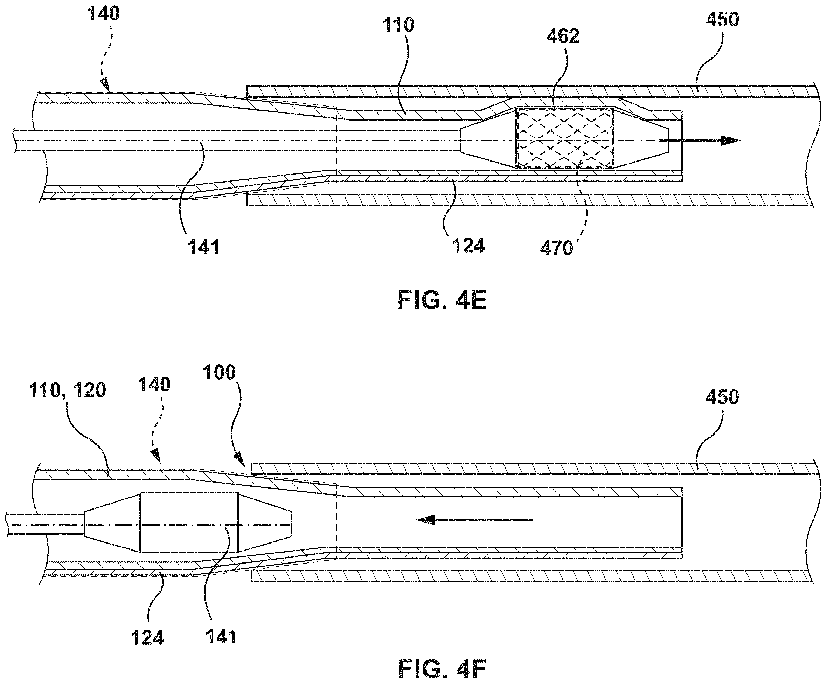

FIGS. 4-4D illustrate an embodiment of a method 400 for accessing a vasculature 450 of a patient and delivering a device 460 towards a desired site in the vasculature using the expandable introducer 100. The device 460 can be, for example, a delivery catheter for delivering a medical device, such as a heart valve prosthesis 470, to a treatment site, such as a native heart valve.

In a step 402 of the method 40, the vasculature 450 of the patient is accessed, such as by using a needle (not shown) to pierce through a wall of a vessel 450, wherein the needle includes a needle lumen. With the needle having gained access to the vessel 450, a guidewire (not shown) is inserted through an opening in a proximal end of the needle lumen. A distal end of the guidewire is advanced through the needle lumen and into the vessel 450 of the patient and a proximal end of the guidewire remains outside of the patient.

In a step 404 of the method 400, the introducer sheath 110 of the expandable introducer 100 and the dilator shaft 210 of the dilator 200 are advanced into the vessel 450 of the patient over the guidewire (not shown). In particular, the dilator 200 is advanced through the introducer 100 such that the dilator shaft 210 is disposed within the introducer sheath 110. The introducer sheath 110 is in the folded configuration and the unexpanded configuration. The introducer sheath 110 and the dilator shaft 210 are advanced to a desired location with the vessel 450, as shown in FIG. 4A. In some embodiments, the introducer sheath 110 and dilator shaft 210 are advanced over a guidewire (not shown). In particular, a proximal end of the guidewire is inserted within the guidewire lumen 222 of the dilator 200 at a distal end of the dilator tip 208. The proximal end of the guidewire is advanced through the lumen 222 until it exits the lumen 222 at the proximal end 204 of the dilator 200. The dilator 200 with the introducer 100 loaded thereon, can then be tracked over the guidewire into the vessel 450 of the patient until the dilator hub 206 of the dilator 200 and the introducer hub 106 of the expandable introducer 100 are snug against the outside of the vessel 450 to establish a hemostatic seal.

In a step 406A of the method 400, the balloon 111 of the introducer sheath 110 of the expandable introducer 100 is partially inflated prior to the dilator 200 being removed. To do this, inflation fluid is introduced to the luer 108 of the introducer hub 106. The inflation fluid advances through the introducer hub 106 and into the inner area 120 of the balloon 111 at its proximal end 114. The inflation fluid entering the inner area 120 of the balloon 111 causes the balloon 111 to partially expand, as shown in FIG. 4B. At this step, the balloon 111 is inflated about 80-95% of maximum inflation. When the balloon 111 is inflated, the one or more ridges 126 on the outer surface 118 of the balloon 111 abuts an interior surface of the vessel 450. The one or more ridges 126 provide spaces between the outer surface 118 of the introducer sheath 110 and the interior surface of the vessel 450 to allow blood to flow and prevent a seal between the vessel 450 and the introducer sheath 110. This, in turn, reduces the insertion force needed to advance the introducer sheath 110 through the vessel 450 of the patient.

The vessel 450 of the patient may include one or more calcium deposits 455 due to calcification of the blood vessel, as shown in FIG. 4C. The calcium deposits 455 protrude within and block or obstruct a portion of the vessel 450, which in turn, blocks or obstructs blood flow within the vessel 450. When the balloon introducer sheath 110 is inflated, the balloon introducer sheath 110 conforms around the calcium deposits 455 and the inflation fluid within the balloon introducer sheath 110 can displace to a different part of the balloon introducer sheath 110, which prevents damage to the vessel 450 of the patient and prevents further obstruction when advancing a device through the balloon introducer sheath 110, explained in more detail below.

In a step 408A of the method 400, the dilator 200 is removed from the vessel 450 of the patient. To do this, the dilator shaft 210 is retracted proximally from the lumen 122 of the expanded introducer sheath 110 until it is removed from the expandable introducer 100 and the vessel 450 of the patient.

In a step 410A of the method 400, a delivery device 460 is advanced through the partially expanded introducer sheath 110. The delivery device 460 may be any delivery device for delivering a prosthesis, such as a heart valve prosthesis 470. The delivery device may include an inner shaft (not shown), an outer shaft 464 and a capsule 462 coupled to a distal end of the outer shaft 464 that encloses the heart valve prosthesis 470 therewithin. In other embodiments, the delivery device may be balloon catheter including a balloon at a distal end thereof, which the heart valve prosthesis 470 crimped onto the balloon. In such a balloon catheter delivery device, a capsule may not be included. In either delivery device, or other similar delivery devices for delivering a heart valve prosthesis, the outer diameter OD3 where the heart valve prosthesis 470 is disposed (i.e., at the balloon or the capsule) is generally larger than the outer diameter of the remainder of the delivery device distal of the handle (not shown) of the delivery device, as shown in FIGS. 4C-4D. For example, the outer diameter OD3 may be in the range of about 8.4 mm to about 10.6 mm. The delivery device 460 may be advanced through the introducer 100 by loading a proximal end of the guidewire into a distal opening of a guidewire lumen of the delivery device 460 and then advancing the delivery device 460 over the guidewire, as known to those skilled in the art, or other methods known to those skilled in the art.

As the delivery device 460 is advanced through the lumen 122 of the partially expanded introducer sheath 110, the capsule 462 (or other enlarged area of the delivery device 460) displaces the inflation fluid within the inner area 120 of the balloon 111 of the introducer sheath 110 such that the wall of the introducer sheath 110 where the introducer sheath 110 encloses the heart valve prosthesis 470 has a thickness that is less than a thickness of the wall of the remainder of the introducer sheath 110, as shown best in FIG. 4D. Because the balloon 111 of the introducer sheath 110 is partially inflated and not fully inflated, the inflation fluid within the inner area 120 of the balloon introducer sheath 110 is able to shift distally or proximally within the inner area 120 of the balloon 111 such that some portions of the balloon introducer sheath 110 may be more inflated that other portions of the balloon introducer sheath 110. In other words, because the outer diameter OD3 of the delivery device 460 at the location of the hearth valve prosthesis 470 (such as at the capsule 462) is greater than the remainder of the delivery device 460, the capsule 462 abuts the interior surface 116 of the balloon 111 and pushes the interior surface 116 towards the exterior surface 118 of the balloon 111. Thus, the inflation fluid, disposed in the inner area 120 in between the interior surface 116 and the exterior surface 118 of the balloon 111, is pushed both proximally and distally away from the section of the balloon introducer sheath 110 that encloses the capsule 462/heart valve prosthesis 470, as indicated by the arrows in FIG. 4D.

FIGS. 4 and 4E illustrate another embodiment of the method 400. As can be seen in the block diagram of the method 400 in FIG. 4, the first and second steps 402, 404 of the method 400 are the same. Therefore, the introducer sheath 110 of the expandable introducer 100 is loaded onto the dilator shaft 210 of the dilator 200 and the assembly is tracked within the vessel 450 of the patient in the same way as previously described. In the method previously described, the balloon introducer sheath 110 is inflated prior to removal of the dilator 200. In this embodiment, the balloon 111 of the introducer sheath 110 is not inflated prior to removal of the dilator 200.

Because the balloon introducer sheath 110 includes a fold 130 and is not inflated prior to the removal of the dilator 200, the expandable introducer 100 further includes a hemostatic jacket 140. The hemostatic jacket 140 is a tubular structure that encloses, or covers, the proximal end 114 of the balloon introducer sheath 110 to prevent blood from flowing outside of the balloon introducer sheath 110. A proximal end of the hemostatic jacket 140 is coupled to the introducer hub 106 and extends distally therefrom. The hemostatic jacket 140 is disposed radially outward from the balloon introducer sheath 110 such that the balloon introducer sheath 110 is disposed within a lumen 141 of the hemostatic jacket 140. The hemostatic jacket 140 may be coupled to the introducer sheath 110 by heat bonding. The hemostatic jacket 140 is not coupled to the introducer sheath 110 along the fold 130 of the balloon introducer sheath 110 such that the fold 130 is able to unfold when the delivery device 460 is advanced therethrough, which will be explained in further detail below. The hemostatic jacket 140 may comprise ePTFE, low durometer PEBAX (about 25D), polyurethane, and/or other expandable materials, and/or any combinations thereof. Such material allows the hemostatic jacket 140 to be low stiffness and minimizes the force needed to expand the balloon introducer sheath 110. In some embodiments, the hemostatic jacket 140 may be about 100 mm to about 125 mm in length such that the distal end of the hemostatic jacket 140 terminated prior to the distal end 112 of the balloon introducer sheath 110, as shown in FIG. 4E.

As such, in a step 406B of the method 400, the dilator 200 is removed from the vessel 450 of the patient. To do this, the dilator shaft 210 is retracted proximally from the lumen 122 of the folded, unexpanded balloon introducer sheath 110 until it is removed from the expandable introducer 100 and the vessel 450 of the patient.

In a step 408B of the method 400, a delivery device 460 is advanced through the folded, unexpanded introducer sheath 110. In this example, the delivery device 460 is the same as shown and described above with respect to FIG. 4C. The delivery device 460 may be loaded onto the guidewire and advanced through the lumen 122 of the folded, unexpanded introducer sheath 110 starting at its proximal end 144. As the delivery device 460 is advanced through the lumen 122 of the folded, unexpanded introducer sheath 110, the fold 130 of the introducer sheath 110 unfolds to the unfolded configuration in response to the delivery device 460/470 being advanced therethrough, as shown in FIG. 4E. Accordingly, as the introducer sheath 110 transitions from the folded configuration to the unfolded configuration, the outer diameter of the introducer sheath 110 increases to accommodate the delivery device 460 being advanced therethrough. As stated previously, the hemostatic jacket 140 does not prevent the balloon introducer sheath 110 from transitioning to the unfolded configuration. In addition, the balloon introducer sheath 110 is not inflated at this step.

In a step 412 of the method 400, to remove the delivery device 460 from the vessel 450 of the patient, the balloon 111 of the introducer sheath 110 is fully inflated prior to proximal retraction of the delivery device 460, as shown in FIG. 4F. The balloon 111 of the introducer sheath 110 is fully inflated prior to removal of the delivery device 460 to provide column strength and rigidity to the introducer sheath 110 to ease removal of the delivery device. To fully inflate the balloon 111, inflation fluid is introduced at the luer 108 of the introducer hub 106. As stated previously, the inflation fluid travels from the introducer hub 106 and enters the inner area 120 of the balloon 111 of the introducer sheath 110 at its proximal end 114. The inflation fluid enters the inner area 120 of the balloon introducer sheath 110 until the balloon introducer sheath 110 expands to full expansion, *i.e.* 100% of maximum inflation. Next, the delivery device 460 is proximally retracted through the lumen 122 of the introducer sheath 110 until the delivery device 460 is removed from the introducer 100 and the vessel 450 of the patient.

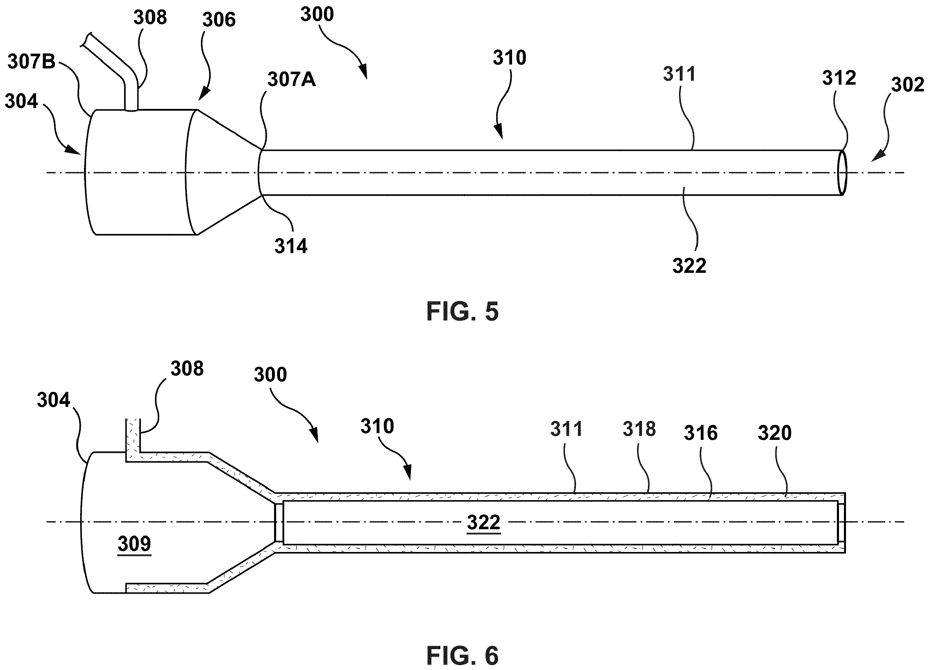

FIGS. 5-10B show an expandable introducer 300 according to embodiments hereof. The expandable introducer 300 includes an introducer hub 306 and an introducer sheath 310. The expandable introducer 300 includes a distal end 302 and a proximal end 304. The introducer sheath 310 is coupled to a distal end 307A of the introducer hub 306 and extends distally therefrom.

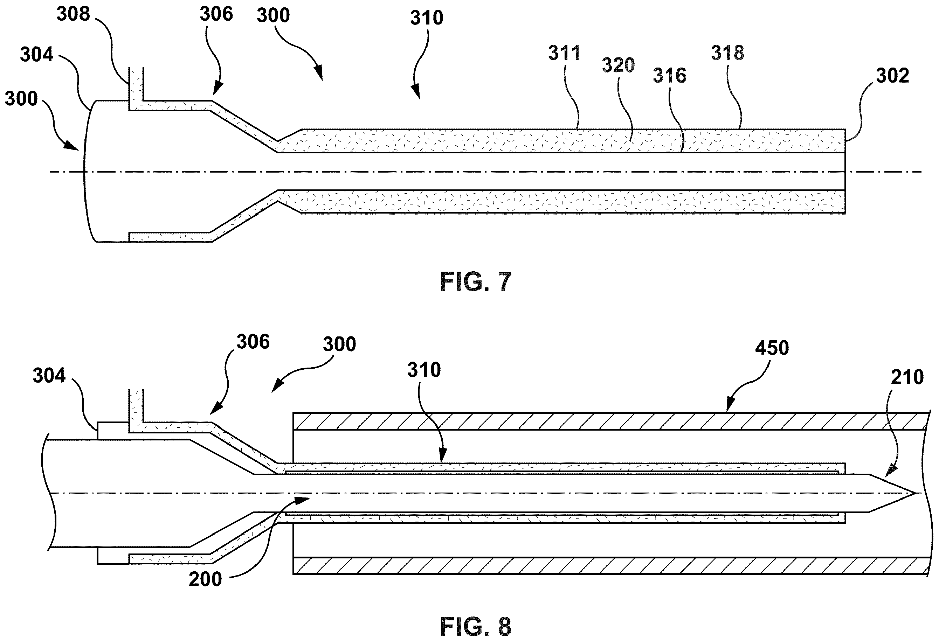

The introducer hub 306 includes the distal end 307A, a proximal end 307B, and a passageway 309 extending from the proximal end 307B to the distal end 307A of the introducer hub 306. The passageway 309 is sized and shaped to accommodate a delivery system therethrough, as described in more detail below. The introducer hub 306 may further include a side port or luer 308 disposed on an outer surface of the introducer hub 306 that is configured to inflate the introducer sheath 310 when in use, which is described in further detail below. In other words, the luer 308 may be utilized for infusing saline or other inflation fluids into the balloon introducer sheath 310, as shown in FIGS. 6 and 7. The introducer hub 306 may further include a seal (not shown) disposed within the passageway 309 to seal around a dilator shaft of a dilator (shown in FIG. 8) or the delivery system, and prevent blood flow out of a patient's vessel when the introducer 300 is in use.

The introducer sheath 310 includes a substantially tubular-shaped balloon 311 defining a lumen 322 of the introducer sheath 310. The introducer sheath 110 may also be referred to as a balloon introducer sheath. The balloon 311 includes a distal end 312, a proximal end 314, an interior surface 316 and an exterior surface 318, as best shown in FIGS. 6 and 7, which also define the distal end, proximal end, interior surface, and exterior surface of the introducer sheath 310. A space between the interior surface 316 and the exterior surface 318 of the balloon 311 defines an inner area 320 of the balloon 311, which defines a wall 321 of the introducer sheath 310. The inner area 320 of the balloon 311 may be inflated to expand the wall 321 of the introducer sheath 310, *i.e.* increase the thickness of the wall 321 of the introducer sheath 310, as described in further detail below. The interior surface 316 of the balloon 311 defines the lumen 322 of the introducer sheath 310 that extends from the distal end 312 to the proximal end 314 of the introducer sheath 310. The lumen 322 is sized and shaped to accommodate a dilator 200 or a delivery system 460 therethrough, as explained in further detail below.

Although not shown with respect to the embodiment of FIGS. 5-10B, at least a portion of the balloon 311, and hence the introducer sheath 310, may be configured to be folded into a folded, unexpanded configuration during insertion within a vessel of a patient and can be unfolded to an unfolded, expanded configuration via inflation of the balloon 311, as explained above with respect to FIGS. 2C-2D. The introducer sheath 310 may have a longitudinal length of about 370 mm and may comprise low friction flexible materials such as, but not limited to, polyvinyl chloride (PVC), polyethylene terephthalate (PET), and/or any other materials known to those skilled in the art.

Although not shown in FIGS. 5-10B, the introducer sheath 310 may also include a spine fused to the exterior surface 318 of the balloon 311, as described above, to provide column strength to the introducer sheath 310.

Although also not shown with respect to FIGS. 5-10B, the exterior surface 318 of the balloon 311 of the introducer sheath 310 may include a plurality of ridges or protrusions extending outwardly from the exterior surface 318, as described above. The ridges are configured to prevent sealing the balloon 311 to the vessel of the patient. The ridges are also configured to reduce the area of contact between the balloon 311 and the vessel of the patient, thereby reducing friction between the balloon 311 and the vessel of the patient to reduce the necessary insertion force when advancing the introducer sheath 310 within the vessel of the patient.

FIG. 6 shows the introducer 300 with the introducer sheath 310 in the uninflated configuration. FIG. 7 shows the introducer 300 with the introducer sheath 310 in the inflated configuration, with an inflation fluid delivered into the inner area 320 of the balloon 311 of the introducer sheath 310. As can be seen in FIG. 7, when the balloon 311 is inflated without outside influence (i.e., not within a vessel wall), both the exterior surface 318 and the interior surface 316 expand.

With the introducer 300 described, FIGS. 8-10B illustrate an embodiment of a method for accessing a vasculature 450 of a patient and delivering a device 460 towards a desired site in the vasculature using the expandable introducer 300. The device 460 can be, for example, a delivery catheter for delivering a medical device, such as a heart valve prosthesis 470, to a treatment site, such as a native heart valve.

In a step of the method, the vasculature 450 of the patient is accessed, such as by using a needle (not shown) to pierce through a wall of a vessel 450, wherein the needle includes a needle lumen. With the needle having gained access to the vessel 450, a guidewire (not shown) is inserted through an opening in a proximal end of the needle lumen. A distal end of the guidewire is advanced through the needle lumen and into the vessel 450 of the patient and a proximal end of the guidewire remains outside of the patient.

In another step of the method, the introducer sheath 310 of the expandable introducer 300 and the dilator shaft 210 of the dilator 200 are advanced into the vessel 450 of the patient over the guidewire (not shown). In particular, the dilator 200 is advanced through the introducer 300 such that the dilator shaft 210 is disposed within the introducer sheath 310. The introducer sheath 310 is in the unexpanded (uninflated) configuration and in the folded configuration of the introducer sheath 310 includes a folded configuration. The introducer sheath 310 and the dilator shaft 210 are advanced to a desired location with the vessel 450, as shown in FIG. 8. In some embodiments, the introducer sheath 310 and dilator shaft 210 are advanced over a guidewire (not shown). In particular, a proximal end of the guidewire is inserted within the guidewire lumen 222 of the dilator 200 at a distal end of the dilator tip 208. The proximal end of the guidewire is advanced through the lumen 222 until it exits the lumen 222 at the proximal end 204 of the dilator 200. The dilator 200 with the introducer 300 loaded thereon, can then be tracked over the guidewire into the vessel 450 of the patient until the dilator hub 206 of the dilator 200 and the introducer hub 306 of the expandable introducer 300 are snug against the outside of the vessel 450.

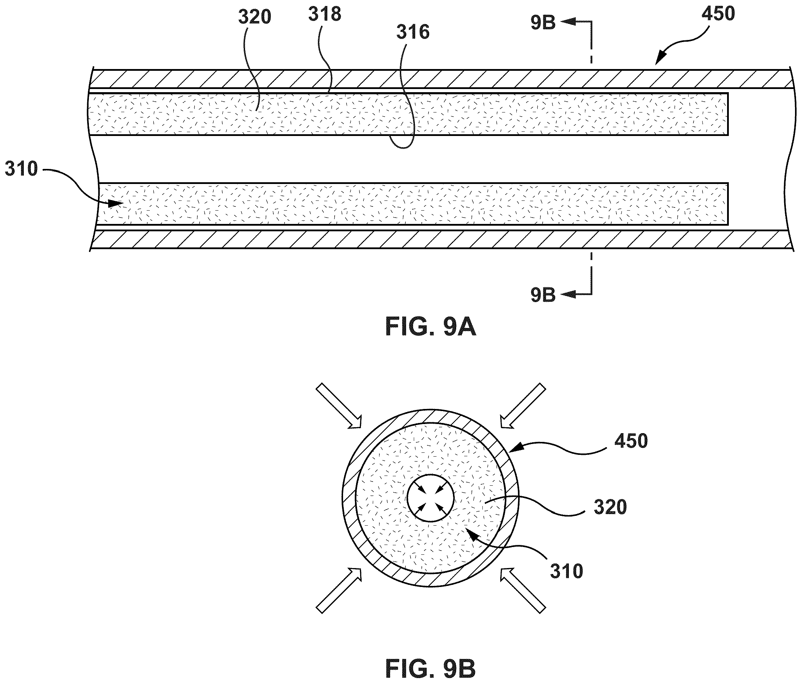

In another step of the method, the balloon 311 of the introducer sheath 310 of the expandable introducer 300 is partially inflated prior to the dilator 200 being removed. To do this, inflation fluid is introduced to the luer 308 of the introducer hub 306. The inflation fluid advances through the introducer hub 306 and into the inner area 320 of the balloon 311 at its proximal end 314. The inflation fluid entering the inner area 320 of the balloon 311 causes the balloon 311 to partially expand, as shown in FIG. 9A. At this step, the balloon 311 may be inflated to about 80-95% of maximum inflation. When the balloon 311 is inflated, the outer surface 318 of the balloon 311 abuts an interior surface of the vessel 450. The vessel 450 applies an inward pressure on the introducer sheath 310, as shown in FIG. 9B, such that the exterior surface 318 of the balloon 311 does not expand to its full diameter that was shown in FIG. 7.

In another step of the method, the dilator 200 is removed from the vessel 450 of the patient. To do this, the dilator shaft 210 is retracted proximally from the lumen 322 of the expanded introducer sheath 310 until it is removed from the expandable introducer 300 and the vessel 450 of the patient.

In another step of the method, a delivery device 460 is advanced through the partially expanded introducer sheath 310. The delivery device 460 may be any delivery device for delivering a prosthesis, such as a heart valve prosthesis 470. The delivery device may include an inner shaft (not shown), an outer shaft 464 and a capsule coupled to a distal end of the outer shaft 464 that encloses the heart valve prosthesis 470 therewithin. In other embodiments, the delivery device may be balloon catheter including a balloon 466 at a distal end thereof, which the heart valve prosthesis 470 crimped onto the balloon. In such a balloon catheter delivery device, a capsule may not be included. In either delivery device, or other similar delivery devices for delivering a heart valve prosthesis, the outer diameter OD3 where the heart valve prosthesis 470 is disposed (i.e., at the balloon or the capsule) is generally larger than the outer diameter of the remainder of the delivery device distal of the handle (not shown) of the delivery device. For example, the outer diameter OD3 may be in the range of about 8.4 mm to about 10.6 mm. The delivery device 460 may be advanced through the introducer 300 by loading a proximal end of the guidewire into a distal opening of a guidewire lumen of the delivery device 460 and then advancing the delivery device 460 over the guidewire, as known to those skilled in the art, or other methods known to those skilled in the art.

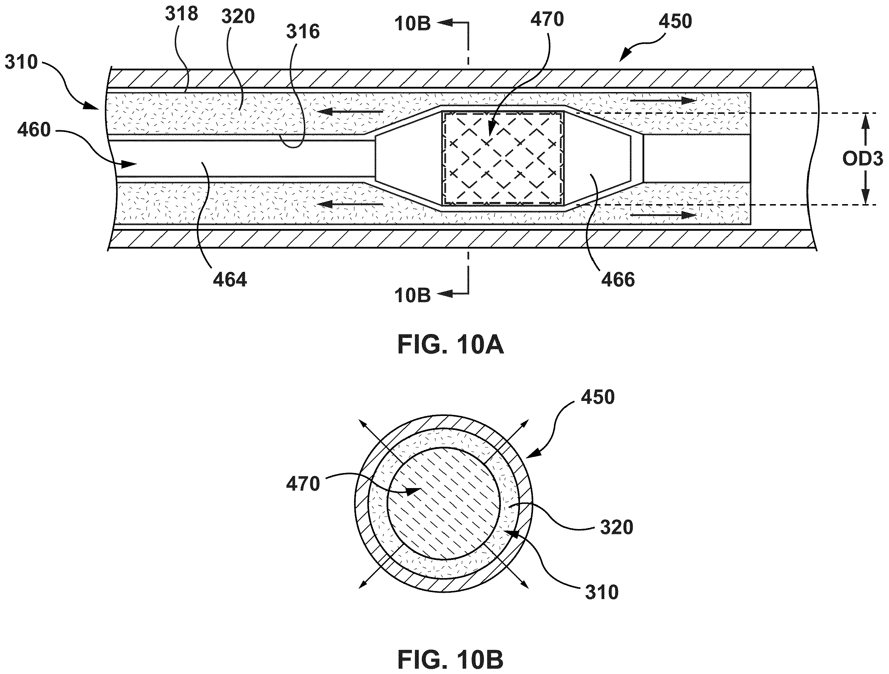

As the delivery device 460 is advanced through the lumen 322 of the partially expanded introducer sheath 310, the heart valve prosthesis 470 disposed on the balloon 464 (or other enlarged area of the delivery device 460 such as a capsule) displaces the inflation fluid within the inner area 320 of the balloon 311 of the introducer sheath 310 such that the wall of the introducer sheath 310 where the introducer sheath 310 encloses the heart valve prosthesis 470 has a thickness that is less than a thickness of the wall of the remainder of the introducer sheath 110, as shown best in FIGS. 10A and 10B. Because the balloon 311 of the introducer sheath 310 is partially inflated and not fully inflated, the inflation fluid within the inner area 320 of the balloon introducer sheath 310 is able to shift distally or proximally within the inner area 320 of the balloon 311 such that some portions of the balloon introducer sheath 310 may be more inflated than other portions of the balloon introducer sheath 310. In other words, because the outer diameter OD3 of the delivery device 460 at the location of the hearth valve prosthesis 470 (such as at the ballon 464) is greater than the remainder of the delivery device 460, the balloon 464 abuts the interior surface 316 of the balloon 311 and pushes the interior surface 316 towards the exterior surface 318 of the balloon 311, as shown in FIG. 10B. Thus, the inflation fluid disposed in the inner area 320 in between the interior surface 416 and the exterior surface 418 of the balloon 411, is pushed both proximally and distally away from the section of the balloon introducer sheath 410 that encloses the balloon 464/heart valve prosthesis 470, as indicated by the arrows in FIG. 10A.

While the devices and methods have been disclosed herein as used for delivery of a heart valve prosthesis, they may be used for delivery of any catheter-based device (e.g., stent graft, stent, balloon, etc.) and may be used in any suitable blood vessel.

It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single device or component for purposes of clarity, it should be understood that the techniques of this disclosure may be performed by a combination of devices or components.

The following examples are illustrative of the techniques described therein.

Example 1. A method of delivering a prosthesis within a vasculature of a patient, the method comprising: advancing an introducer sheath of an expandable introducer into a vessel of the patient, the introducer sheath being in a folded configuration, the introducer sheath including a balloon having an interior surface defining a central lumen of the introducer sheath, an exterior surface, and an inner area between the interior surface and the exterior surface; partially inflating the balloon of the balloon introducer sheath by introducing inflation fluid into the inner area of the balloon; advancing a delivery device through the central lumen of the introducer sheath, wherein advancing the delivery device causes the inflation fluid within the balloon to displace.

Example 2. The method of Example 1, further comprising: after the delivery device has been advanced through the central lumen of the introducer sheath, fully inflating the balloon of the introducer sheath; and with the balloon fully inflated, retracting the delivery device proximally through the central lumen of the introducer sheath.

Example 3. The method of Example 1, further comprising inserting a dilator into the expandable introducer prior to advancing the introducer sheath into the patient's vessel, wherein advancing the introducer sheath into the patient's vessel comprises advancing the introducer sheath and a dilator shaft of the dilator into the patient's vessel.

Example 4. The method of Example 3, further comprising removing the dilator from the expandable introducer prior to advancing the delivery device through the central lumen of the introducer sheath.

Example 5. The method of Example 1, wherein inflating the balloon causes the introducer sheath to unfold to an unfolded configuration.

Example 6. The method of Example 1, wherein advancing the delivery device through the central lumen of the introducer sheath causes the introducer sheath to unfold to an unfolded configuration.

Example 7. A method of delivering a prosthesis within a vasculature of a patient, the method comprising: advancing an introducer sheath of an expandable introducer into a vessel of the patient, the introducer sheath being in a folded configuration, the introducer sheath including a balloon having an interior surface defining a central lumen of the introducer sheath, an exterior surface, and an inner area between the interior surface and the exterior surface; advancing a delivery device through the central lumen of the introducer sheath, wherein advancing the delivery device causes introducer sheath to unfold to an unfolded configuration.

Example 8. The method of claim 7, further comprising: after the delivery device has been advanced through the central lumen of the introducer sheath, fully inflating the balloon of the introducer sheath; and with the balloon fully inflated, retracting the delivery device proximally through the central lumen of the introducer sheath.

Example 9. The method of Example 7, further comprising inserting a dilator into the expandable introducer prior to advancing the introducer sheath into the patient's vessel, wherein advancing the introducer sheath into the patient's vessel comprises advancing the introducer sheath and a dilator shaft of the dilator into the patient's vessel.

Example 10. The method of Example 9, further comprising removing the dilator from the expandable introducer prior to advancing the delivery device through the central lumen of the introducer sheath.

Example 11. An expandable introducer for delivering a medical device, the expandable introducer comprising: an introducer hub; and an introducer sheath coupled to the introducer hub and extending distally therefrom, wherein the introducer sheath includes a balloon having an interior surface defining a central lumen of the introducer sheath, an exterior surface, and an inner area between the interior surface and the exterior surface, the inner area configured to be inflated, wherein at least a portion of the introducer sheath includes a fold such that the introducer sheath includes a folded configuration and an unfolded configuration, wherein the balloon introducer sheath transitions from the folded configuration to the unfolded configuration in response to a delivery device being advanced therethrough or to the balloon introducer sheath being inflated.

Example 12. The expandable introducer of Example 11, wherein the balloon includes one or more ridges on the exterior surface thereof.

Example 13. The expandable introducer of Example 11, wherein the introducer sheath is configured to be loaded onto a dilator in the folded configuration prior to insertion within a vessel of a patient.

Example 14. The expandable introducer of Example 11, wherein the balloon of the introducer sheath is configured to be partially inflated prior to advancing a delivery device therethrough.

Example 15. The expandable introducer of Example 11, wherein with the balloon partially inflated, wherein advancement of the delivery device through the balloon introducer sheath causes inflation fluid within the balloon introducer sheath to displace.

Example 16. The expandable introducer of Example 11, wherein the balloon is configured to be fully inflated prior to retraction of the delivery device from the introducer sheath.

Example 17. The expandable introducer of Example 11, wherein an outer diameter of the introducer sheath in the unfolded configuration is greater than an outer diameter of the balloon introducer sheath in the folded configuration.

Example 18. The expandable introducer of Example 11, wherein the expandable introducer further includes a spine coupled to the balloon introducer sheath.

Example 19. The expandable introducer of Example 18, wherein the spine is a rigid polymer.

Example 20. The expandable introducer of Example 18, wherein the spine runs longitudinally along the balloon and is disposed opposite the fold.

Further disclosed herein is an expandable introducer including a hub and an introducer sheath extending distally from the hub. The introducer sheath includes a balloon having an interior surface defining a central lumen of the introducer sheath, an exterior surface, and an inner area between the interior surface and the exterior surface. The introducer sheath includes a folded configuration and an unfolded configuration. The introducer sheath is configured to be advanced into a vessel of a patient in the folded configuration. The balloon may be partially inflated prior to advancement of a delivery device through the introducer sheath such that the delivery device displaces inflation fluid in the partially inflated balloon. The introducer sheath may be unfolded by inflation of the balloon or by advancement of the delivery device through the introducer sheath. The balloon is configured to be fully inflated prior to retraction of the delivery device through the introducer sheath.

## Claims

1. An expandable introducer for delivering a medical device, the expandable introducer comprising:
an introducer hub; and
an introducer sheath coupled to the introducer hub and extending distally therefrom, wherein the introducer sheath includes a balloon having an interior surface defining a central lumen of the introducer sheath, an exterior surface, and an inner area between the interior surface and the exterior surface, the inner area configured to be inflated, wherein at least a portion of the introducer sheath includes a fold such that the introducer sheath includes a folded configuration and an unfolded configuration,
wherein the balloon introducer sheath transitions from the folded configuration to the unfolded configuration in response to a delivery device being advanced therethrough or to the balloon introducer sheath being inflated.

2. The expandable introducer of claim 1, wherein the balloon includes one or more ridges on the exterior surface thereof.

3. The expandable introducer of claim 1 or claim 2, wherein the introducer sheath is configured to be loaded onto a dilator in the folded configuration prior to insertion within a vessel of a patient.

4. The expandable introducer of any one of claims 1 to 3, wherein the balloon of the introducer sheath is configured to be partially inflated prior to advancing a delivery device therethrough.

5. The expandable introducer of any one of claims 1 to 4, wherein with the balloon partially inflated, wherein advancement of the delivery device through the balloon introducer sheath causes inflation fluid within the balloon introducer sheath to displace.

6. The expandable introducer of any one of claims 1 to 5, wherein the balloon is configured to be fully inflated prior to retraction of the delivery device from the introducer sheath.

7. The expandable introducer of any one of claims 1-6, wherein an outer diameter of the introducer sheath in the unfolded configuration is greater than an outer diameter of the balloon introducer sheath in the folded configuration.

8. The expandable introducer of any one of claims 1 to 7, wherein the expandable introducer further includes a spine coupled to the balloon introducer sheath.

9. The expandable introducer of claim 8, wherein the spine is a rigid polymer.

10. The expandable introducer of claim 8 or claim 9, wherein the spine runs longitudinally along the balloon.

11. The expandable introducer of any one of claims 8 to 10, wherein the spine is disposed opposite the fold.
